# EUROPEAN PATENT APPLICATION

(11) **EP 1 980 550 A1**
(43) Date of publication of application: **15.10.2008**
(21) Application number: 06843257.4
(22) Date of filing: 26.12.2006
(51) Int. Cl.: C07C 37/68, C07C 39/16

(54) **PROCESS FOR PRODUCING HIGH-QUALITY GRANULAR BISPHENOL A**

(30) Priority: 27.01.2006 JP 2006019787
(71) Applicant: Idemitsu Kosan Co., Ltd., Chiyoda-ku Tokyo 100-8321 (JP)
(72) Inventor: YOSHITOMI, Kazuyuki, Ichihara-shi, Chiba 2990193 (JP); MASUDA, Shuichi, Ichihara-shi, Chiba 2990193 (JP); KODAMA, Masahiro, Ichihara-shi, Chiba 2990193 (JP)
(74) Representative: Vossius & Partner
(86) International application number: PCT/JP2006/325873
(87) International publication number: WO 2007/086226

(57) **Abstract**

A process for producing high-quality granular bisphenol A is provided. In the process, granular bisphenol A (2,2-bis(4-hydroxyphenyl)propane) is contacted to a metal-scavenging apparatus placed in a granulation step or a subsequent step thereof so as to remove efficiently from granular bisphenol A metal powder that have come into the bisphenol Ain a granulation step or a subsequent step thereof or that have resulted from regular or irregular overhaul inspection or repairing of various devices in the a production apparatus.

## Description

### [Technical Field]

The present invention relates to a process for producing high-quality granular bisphenol A (2,2-bis(4-hydroxyphenyl)propane). More specifically, the present invention relates to a process for producing high-quality granular bisphenol A characterized in that the iron content of the granular bisphenol A is reduced by collecting metallic powder contained therein with a metal-scavenging apparatus placed in a granulation step or a subsequent step thereof.

### [Background Art]

Bisphenol A has been widely used conventionally as a raw material for polymers such as polycarbonate, epoxy resin, and polyacrylate. Among these polymers obtained from bisphenol A, polycarbonate has a remarkable growing demand in recent years as an optical material for use in an application such as optical disk substrates.
Polycarbonate used as the optical material is required to reduce infinitely small the amount of foreign substances contained therein and is also required to be infinitely clear color. The same requests are undoubtedly also directed to bisphenol A that is a raw material of polycarbonate.

As a process for producing bisphenol A in which product quality is improved by removing foreign substances, Patent Document 1 discloses a method for producing bisphenols in which phenols and ketones are reacted to form bisphenols and the resulting liquid bisphenols or a mixed liquid of the bisphenols and phenols are filtered with a sintered metal filter. Patent Document 2 discloses a method for producing bisphenol A in which foreign substances are removed by placing a glass fiber filter in the production process of bisphenol A and allowing a bisphenol A solution to flow through the glass fiber filter.

Generally, bisphenol A is produced through the following steps: (A) acetone and an excess amount of phenol are condensed in the presence of an acidic catalyst to obtain a reaction mixture liquid; (B) the reaction mixture liquid obtained in the step (A) is concentrated; (C) an adduct of bisphenol A and phenol is crystallized and separated from the concentrated residual liquid obtained in the step (B); (D) the adduct of bisphenol A and phenol crystallized and separated in the step (C) is dissolved in a phenol-containing solution; (E) an adduct of bisphenol A and phenol is crystallized and separated from the solution obtained in the step (D) and, depending on the situation, an operation of further dissolving the adduct in a phenol-containing solution followed by crystallization and separation is repeated at least one time; (F) the adduct of bisphenol A and phenol crystallized and separated in the step (E) is heated and melted, and then phenol is distilled out; and (G) the bisphenol A melt obtained in the step (F) is cooled, solidified, and granulated into granular bisphenol A. The granular bisphenol A obtained in the step (G) conveyed to a product silo and stored therein or delivered.

A conveyor machine such as a rotary valve and a bucket conveyor is generally used to convey the granular bisphenol A to the product silo. The internal parts of these conveyor machines include stainless-steel or iron made parts. Stainless-steel or iron powder produced by metal-to-metal abrasion inside the conveyor machines is contained in the granular bisphenol A.
Therefore, even though foreign substances are removed from the bisphenol A solution with a filter or the like in each step described above preceding to the granulation step, if the metal powder produced in the granulation step or a subsequent step thereof is contained as a foreign substance in the product granular bisphenol A, a product-quality problem will be brought about on a delivered site, that is, in the production process of polycarbonate. The same problem will be met also in a facility (receiving silo or the like) receiving the bisphenol A in the production process of polycarbonate.
Further, in the case where various instruments inside of a bisphenol A production plant are regularly or irregularly subjected to an open-up inspection or repair, the parts inside of the instruments are exposed to the open air and occur rust (iron oxide). If the operation is restarted while the rust remains untouched, metal powder is produced anomalously, thereby bringing about a problem of requiring a troublesome treatment of product bisphenol A, which contains a large amount of the metal powder produced upon restarting.

Patent Document 1: Japanese Patent Laid-Open Publication No. 11-180920,
Patent Document 2: Japanese Patent Laid-Open Publication No. 2000-327614.

### [Disclosure of the Invention]

### [Problems to be Solved by the Invention]

The present invention has been made to address the problems brought about under the aforementioned circumstances. It is an object of the present invention to provide a process for producing high-quality granular bisphenol A by removing efficiently from granular bisphenol A metal powder that have come into the bisphenol A in a granulation step or a subsequent step thereof or that have resulted from regular or irregular overhaul inspection or repairing of various devices in the a production apparatus.

### [Means for Solving the Problems]

The present inventors have made intensive studies to achieve the aforementioned objective. As a result, it has been found to be achieved by contacting granular bisphenol A to a metal scavenging apparatus placed in a granulation step or a subsequent step thereof and collecting metal powder contained in the granular bisphenol A. Based on this finding, the present invention has been accomplished.

Namely the present invention provides:
(1) A Process for producing high-quality granular bisphenol A including contacting granular bisphenol A (2,2-bis(4-hydroxyphenyl)propane) to a metal-scavenging apparatus placed in a granulation step or a subsequent step thereof and collecting metal powder contained in the granular bisphenol A so as to reduce an iron content of the granular bisphenol A;
(2) The process for producing high-quality granular bisphenol A as described in (1), wherein the metal-scavenging apparatus is placed in a pipe drawing out granular bisphenol A at the bottom of a granulation tower, in an inlet or an outlet of a product silo, or in an inlet or an outlet of a receiving silo for granular bisphenol A on a delivered site;
(3) The process for producing high-quality granular bisphenol A as described in (1) or (2), wherein the metal powder is stainless-steel powder and/or iron powder;
(4) The process for producing high-quality granular bisphenol A as described in any one of (1) to (3), wherein the iron content of the granular bisphenol A after the granulation step is less than 1 ppm by mass;
(5) The process for producing high-quality granular bisphenol A as described in any one of (1) to (4), wherein the metal-scavenging apparatus is provided with a magnet;
(6) The process for producing high-quality granular bisphenol A as described in (5), wherein the surface magnetic flux density of the magnet is 0.7 tesla (T) or more; and
(7) The process for producing high-quality granular bisphenol A as described in any one of (1) to (6), wherein the metal-scavenging apparatus is a magnet bar placed perpendicularly to the flow direction of granular bisphenol A conveyed.

### [Brief Description of Drawings]

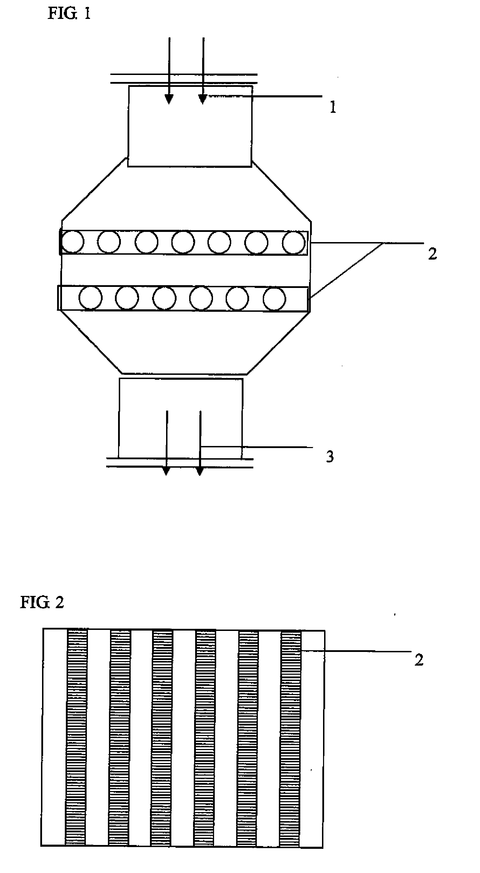

FIG. 1 is a schematic view illustrating a vertical section of a metal-scavenging apparatus according to the present invention.
FIG. 2 is a schematic plan view of a magnet bar serving as a metal-scavenging apparatus according to the present invention.

### [Explanation of Reference Numerals]

1: Inlet of a metal-scavenging apparatus for granular bisphenol A,
2: Magnet bar, and
3. Outlet of a metal-scavenging apparatus for granular bisphenol A.

### [Best Mode for Carrying Out the Invention]

In the process for producing high-quality granular bisphenol A according to the present invention, granular bisphenol A is contacted to a metal-scavenging apparatus placed in a granulation step or a subsequent step thereof so as to collect metal powder, thereby reducing the iron content in the granular bisphenol A.

Generally, bisphenol A is produced through the following steps: (A) acetone and an excess amount of phenol are condensed in the presence of an acidic catalyst to obtain a reaction mixture solution; (B) the reaction mixture solution obtained in the step (A) is concentrated; (C) an adduct of bisphenol A and phenol is crystallized and separated from the concentrated residual liquid obtained in the step (B); (D) the adduct of bisphenol A and phenol crystallized and separated in the step (C) is dissolved in a phenol-containing solution; (E) an adduct of bisphenol A and phenol is crystallized and separated from the solution obtained in the step (D) and, depending on the situation, an operation of further dissolving the adduct in a phenol-containing solution followed by crystallization and separation is repeated at least one time; (F) the adduct of bisphenol A and phenol crystallized and separated in the step (E) is heated and melted, and then phenol is distilled out; and (G) the bisphenol A melt obtained in the step (F) is cooled, solidified, and granulated into granular bisphenol A. The granular bisphenol A obtained in the step (G) conveyed to a product silo and stored therein or delivered.
Hereinafter, each step will be explained.

### Step (A):

In the step (A), an excess amount of phenol is condensed with acetone to form bisphenol A in the presence of an acidic catalyst. As the acidic catalyst, may be used an acid ion-exchange resin. The acid ion-exchange resin is not particularly limited, but a conventional catalyst used for the production of bisphenol A may be used. In particular, considering catalyst activity, a sulfonic acid cation-exchange resin is suitably used.
The sulfonic acid cation-exchange resin is not particularly limited as long as it is a strongly acidic cation-exchange resin that has a sulfonic acid group. Examples of the sulfonic acid type cation-exchange resin may include sulfonated styrene/divinylbenzene copolymer, sulfonated cross-linked styrene polymer, phenol formaldehyde/sulfonic acid resin, benzene formaldehyde/sulfonic acid resin, and the like. These may be used solely or two or more kinds in combination.
In the production method, along with the acid ion-exchange resin, mercaptans serving as an auxiliary catalyst are usually used in combination. The mercaptans are a compound having a free SH group in the molecule. The mercaptans may include alkyl mercaptans or alkyl mercaptans having at least one kind of substitution group such as carboxyl group, amino group, and hydroxyl group, including, for example, mercapto carboxylic acids, aminoalkane thiols, mercapto alcohols, or the like.

Examples of the mercaptans may include an alkyl mercaptan such as metyl mercaptan, ethyl mercaptan, n-butyl mercaptan, and n-octyl mercaptan; a thiocarboxylic acid such as thio glycolic acid and β-mercapto propionic acid; an aminoalkane thiol such as 2-aminoethane thiol; a mercapto alcohol such as mercapto ethanol; and the like. Among these, alkyl mercaptan is particularly suitable considering the effect as an auxiliary catalyst. These mercaptans may be used solely or two or more kinds in combination.
These mercaptans may be fixed on the aforementioned acid ion-exchange resin so as to serve as an auxiliary catalyst.
The used amount of the mercaptans is selected in the range of generally from 0.1 to 20 mol % and preferably from 1 to 10 mol %.
The ratio of phenol to acetone used is not particularly limited, but it is desirable that the amount of unreacted acetone is limited as small as possible considering easiness of purification of the produced bisphenol A or from the economical viewpoint. Therefore, it is advantageous that phenol is used in excess over the stoichiometric amount. Usually, phenol is used in an amount of 3 to 30 mol, and preferably 5 to 15 mol, per one mol of acetone.
Further, in the bisphenol A production, a reaction solvent is not generally needed except the case where the viscosity of the reaction liquid is too high or the case where the reaction is disturbed by solidification at low temperatures.
The condensation of phenol and acetone may be performed in a batch-wise or a continuous process, but a fixed-bed continuous process is desirable where acetone and phenol are reacted while supplying continuously phenol, acetone, and mercaptans (in the case where mercaptans are not fixed on an acid ion-exchange resin). In this process, the reactor may be one or two or more in series, but industrially it is particularly advantageous that two or more reactors loaded with the acid ion-exchange resin are connected in series and a fixed-bed multi-stage continuous process is applied.

Reaction conditions applied to the fixed-bed continuous process are explained.
Firstly, the molar ratio of acetone/phenol is selected in the range of usually from 1/30 to 1/3 and particularly from 1/15 to 1/5. When the molar ratio is smaller than 1/30, the reaction rate possibly becomes low. At a molar ratio of larger than 1/3, the amount of impurities produced becomes large, thereby the selectivity for bisphenol A is likely to be lowered.
On the other hand, in the case where mercaptans are not fixed on the acid ion-exchange resin, the molar ratio of mercaptans/acetone is selected in the range of usually from 0.1/100 to 20/100 and preferably from 1/100 to 10/100. When the molar ratio is smaller than 1/100, possibly an effect of enhancing the reaction rate or the selectivity for bisphenol A is not exerted sufficiently. At a molar ratio of larger than 20/100, the amount increased seems not to pay for the increase of the effect.
The reaction temperature is selected in the range of usually from 40 to 150°C and preferably from 60 to 110°C. When the temperature is lower than 40°C, the reaction rate becomes low and the viscosity of the reaction liquid becomes extremely high and is possibly solidified in some cases. At a reaction temperature of higher than 150°C, the reaction becomes difficult to control, the selectivity for bisphenol A (p,p'-form) is lowered, and the acid type ion-exchanger resin serving as a catalyst is possibly decomposed or degraded. Further, the LHSV (Liquid Hourly Space Velocity) of the raw material mixture is selected in the range of usually from 0.2 to 30 hr⁻¹ and preferably from 0.5 to 10 hr⁻¹.
In the production method, it is desirable that the reaction mixture solution thus obtained is firstly filtered with a filter. By filtering with a filter the reaction mixture solution that contains bisphenol A, foreign substances contained in the solution can be removed, so that decomposition and color degradation of bisphenol A can be prevented under high temperature conditions in the subsequent steps.
Post treatments of the reaction mixture solution or post treatments performed after the filtration may include a filtering step with a filter that is accompanied by at least one step selected from a step of dissolving an adduct of bisphenol A and phenol in a phenol-containing solution and a step of crystallizing and separating the adduct from the resulting solution, besides the following steps (B) to (F).

### Step (B):

In the step (B), the reaction mixture solution described above that contains substantially no acid ion-exchange resin is concentrated. In this concentration step, usually firstly, low boiling point substances such as unreacted acetone, by-produced water, and alkyl mercaptans are removed by vacuum distillation in a distillation column. The vacuum distillation is carried out under a pressure of about 6.5 to 80 kPa and at a temperature of about 70 to 180°C. In this occasion, unreacted phenol is azeotroped and a part of it is discharged out of the reaction system together with the low boiling point substances from the head of the distillation column. In the vacuum distillation, it is desirable that the temperature of a heat source used is kept 190°C or lower so as to prevent thermal decomposition of bisphenol A.

After that, a column bottom liquid that is obtained by removing the low boiling point substances from the reaction mixture solution and contains bisphenol A, phenol, and others is subjected to vacuum distillation to distill out phenol and to concentrate bisphenol A. The concentration conditions are not particularly limited, but are usually selected as: about 100 to 170°C of temperature and about 5 to 70 kPa of pressure. When the temperature is lower than 100°C, a high vacuum is needed. At a temperature higher than 170°C, an extra operation of removing heat is needed in the following crystallization step.
Further, the concentration of bisphenol A in the residual concentrated liquid is in the range of preferably from 20 to 50% by mass and more preferably from 20 to 40% by mass. When the concentration is lower than 20% by mass, the recovery of bisphenol A is low. At a concentration higher than 50% by mass, possibly the slurry obtained after crystallization becomes difficult to convey.

### Step (C):

In the step (C), a 1:1 adduct (hereinafter, also referred to as phenol adduct) of bisphenol A and phenol is crystallized and separated from the residual concentrated liquid obtained in the step (B). In this step, firstly, the residual concentrated liquid is cooled to about 40 to 70°C so as to crystallize the phenol adduct and to obtain a slurry. The residual concentrated liquid can be cooled with an external heat-exchanger or by using the method of vacuum-vaporization crystallization in which water is added to the residual concentrated liquid so as to cool it with the latent heat of water vaporization under vacuum. In the vacuum-vaporization crystallization, after water is added in an amount of about 3 to 20% by mass to the residual concentrated liquid, the liquid is crystallized generally at a temperature of about 40 to 70°C under a pressure of about 3 to 13 kPa. When the amount of water added is less than 3% by mass, sufficient heat removal effect is not obtained. When the amount goes over 20% by mass, the solubility of bisphenol A becomes increased.

In the crystallization, when the crystallization temperature is lower than 40°C, possibly the viscosity of the crystallizing liquid becomes increased or the liquid is solidified. At a temperature of higher than 70°C, the solubility of bisphenol A becomes increased.

Then, the slurry that contains the phenol adduct thus crystallized is separated by a known method such as filtration and centrifugal separation into the phenol adduct and a crystallization mother liquid containing reaction by-products.

### Step (D):

In the step (D), the phenol adduct crystallized and separated in the step (C) is dissolved in a phenol-containing solution. The phenol-containing solution used in this step is not particularly limited, but may include the recovery phenol obtained in the concentration step of step (B), the washing liquid for the phenol adduct produced in the crystallization and separation step of step (C), the mother liquid obtained by the solid-liquid separation of the crystallized phenol adduct produced in the present step (D) or a subsequent step thereof or washing liquid for the phenol adduct, and the like.

After the phenol-containing solution is added to the phenol adduct obtained in the step (C), the resulting mixture is heated at about 80 to 110°C so as to dissolve the phenol adduct and to prepare a bisphenol A containing solution having a bisphenol A concentration suitable for crystallization in the following step. The bisphenol A containing solution thus prepared is relatively easy to handle because it has a low viscosity at relatively low temperature. The bisphenol A containing solution is suitable for solid/liquid separation of the crystallized phenol adduct with a filter in the following step.

### Step (E):

In the step (E), the phenol adduct is crystallized and separated from the bisphenol A containing solution obtained in the step (D). Further, depending on the situation, in order to obtain a high-quality product, the operation of dissolving the phenol adduct in the phenol-containing solution, and then crystallizing and separating the phenol adduct is repeated at least one time. The operation of crystallizing and separating the phenol adduct and dissolving the phenol adduct in the phenol-containing solution performed in this step is similar to the operation in the steps (C) and (D).

### Step (F):

In the step (F), the phenol adduct crystallized and separated in the step (E) is heated and melted, and then phenol is distilled out from the phenol adduct. In this step, firstly, the phenol adduct is heated at about 100 to 160°C and melted into a liquid mixture; then, phenol is removed by vacuum distillation so as to recover a bisphenol A melt. The vacuum distillation is carried out usually under a pressure of 1 to 11 kPa and at a temperature of 150 to 190°C. Further, the remaining phenol can be removed by steam stripping.

### Step (G):

In the step (G), the bisphenol A melt obtained in the step (F) is solidified and granulated. The method for granulation may include, for example, a method in which the bisphenol A melt is supplied to a cooling drum to solidify it into flakes, or a spray granulation method in which the bisphenol A melt is sprayed from the head of a granulation tower while solidifying it with a cooling gas blowing from the bottom of the tower.

### Storage and delivering of product granular bisphenol A:

The granular bisphenol A solidified and granulated in the step (G) conveyed to various equipments or facilities including, for example, a delivering facility, a product silo, and the like. A conveyor machine such as a rotary valve and a bucket conveyor can be used.

The metal-scavenging apparatus of the present invention is placed in the step (G) or a subsequent step thereof. Specifically, the placing position of the metal-scavenging apparatus may be in a pipe drawing out the granular bisphenol A from the bottom of the granulation tower, in an inlet or outlet of the product silo, and in an inlet or outlet of a granular bisphenol A receiving silo on a delivered site.
The metal-scavenging apparatus preferably is provided with a magnet. The magnet has a surface magnetic flux density of preferably 0.7 tesla (T) or higher and more preferably 0.9 tesla (T) or higher. When the surface magnetic flux density is less than 0.7 tesla, stainless-steel powder that has weak magnetic property is possibly not easy to collect. Here, the surface magnetic flux density denotes the number of magnetic flux passing through per 1 cm² of the magnet surface. Examples of the magnet may include an industrially conventionally used magnet such as a neodymium rare earth magnet.
The magnet is provided in the metal-scavenging apparatus in such a manner as in a preferred example where an assembly of plural of stainless steel bars each having a magnet inserted therein is provided in the metal-scavenging apparatus and in a more preferred example where plural of the assemblies are provided in a multi-stage so as to enhance the capability of collecting metal powder. The shape of the stainless steel bar is not particularly limited, but a round bar is preferable because the granular bisphenol A is not built up easily. The spacing between the stainless steel bars that are plurally assembled is not particularly limited, but is preferably about 20 times to 100 times of the particle size of the granular bisphenol A from the viewpoint of enhancing the capability of collecting metal powder and preventing clogging.
Further, the magnet bars serving as the metal-scavenging apparatus may be placed in any direction with respect to the flow direction of the granular bisphenol A conveyed. In particular, the magnet bars are placed preferably perpendicularly with respect to the flow direction from the viewpoint of enhancing the capability of collecting metal powder.

Metal powder contained in the granular bisphenol A is removed by passing the granular bisphenol A through the metal-scavenging apparatus having a magnet. As a result, the iron content of the granular bisphenol A can be reduced to less than 1 ppm by mass, more preferably less than 0.2 ppm by mass, and still more preferably less than 0.1 ppm by mass. At an iron content of 1 ppm by mass or more, not only the color of the product bisphenol A becomes worse, but also the color of polycarbonate that is produced by using the bisphenol A as a raw material becomes worse or a problem of foreign substances is brought about.
Note that, the iron content of the granular bisphenol A can be measured by conventional analysis such as the ortho-phenanthroline absorption spectroscopy (JIS K 0102) using a photo spectrometer.

### [Examples]

Hereinafter, the present invention will be described in detail with reference to the following examples, but it should be construed that the present invention is in no way limited to those examples.

### Reference Example

Through a fixed-bed reactor loaded with a catalyst of a cation-exchange resin ("DIAION SK104H" (trade name), manufactured by Mitsubishi Chemical Corporation) whose sulfonic acid groups were partly neutralized by 20 mol% with 2-mercapto ethylamine, phenol and acetone (phenol: 32 t/hr, acetone: 3t/hr) were allowed to flow continuously at an LHSV of 3 hr⁻¹ and react at 75°C. The resulting reaction mixture solution was introduced into a vacuum distillation tower. After acetone, water and others were removed by vacuum distillation at a tower bottom temperature of 170°C under a pressure of 67 kPa-A, phenol was further distilled out by vacuum distillation at a temperature of 130°C under a pressure of 14 kPa-A until bisphenol A was concentrated to 40% by mass, so that a bisphenol A/phenol solution was obtained at a rate of 15 t/hr.
After that, water at a rate of 1 t/hr was added to the bisphenol A/phenol solution having a bisphenol concentration of 40% by mass, and the resulting mixture was kept cool at 50°C under a pressure of 5.3 kPa-A so as to crystallize an adduct of bisphenol A and phenol. In this way, a slurry liquid was obtained at a rate of 16 t/hr.
Then, the slurry liquid was subjected to solid/liquid separation to obtain the adduct of bisphenol A and phenol. Phenol was added to the adduct of bisphenol A and phenol, which was then heated at 90°C to obtain a liquid containing 60% by mass of phenol and 40% by mass of bisphenol A. The liquid was filtered with a filter having 10 pm open pores. Again, the liquid was subjected to the same vacuum-vaporization crystallization as above to obtain a slurry liquid at a rate of 16 t/hr.
Next, the slurry liquid was subjected to solid/liquid separation and washed with 1.6 t/hr of phenol to obtain adduct crystals of bisphenol A and phenol. The resulting adduct crystals were heated and melted at 130°C so as to remove phenol, and then, from the resulting melt, were obtained 4.3 t/hr of granular bisphenol A with an average particle size of 1 mm in a spray granulation tower. The iron content of the resulting bisphenol A was 0.1 ppm by mass. The color as evaluated by visual observation was AHPA 15.

### Comparative Example 1

The granular bisphenol A obtained in the Reference Example were conveyed at 4.3 t/hr to a product silo with a rotary valve (rotating blades: made of stainless steel) and a bucket conveyor (bucket: made of stainless-steel, inside chain: made of iron). No metal-scavenging apparatus was placed at the inlet of the silo. The iron content of the product granular bisphenol A sampled out of the product silo was 1.15 ppm by mass. After the bisphenol A was heated at 220°C for 40 minutes in the air, the color evaluated by visual observation using an APHA standard color was APHA 40.

### Example 1

The granular bisphenol A obtained in the Reference Example were conveyed at 4.3 t/hr to a product silo with a rotary valve (rotating blades: stainless steel made) and a bucket conveyor (bucket: stainless-steel made, inside chain: iron made). A metal-scavenging apparatus was placed at the inlet of the silo. The metal-scavenging apparatus was made of two lines (spacing between lines was 60 mm) of a magnet bar array. Each magnet bar was made of a stainless steel pipe 25 mm in diameter and 40 cm long having a magnet (neodymium rare earth metal, surface magnetic flux: 0.9 tesla) inserted therein. The spacing between the stainless steel pipes was 50 mm. The iron content of the product granular bisphenol A sampled at the outlet of the product silo was 0.1 ppm by mass. After the bisphenol A was heated at 220°C for 40 minutes in the air, the color evaluated by visual observation using an APHA standard color was APHA 15.

### Example 2

Granular bisphenol A were produced similarly to Example 1, except that a metal-scavenging apparatus with a magnet having a surface magnetic flux of 0.7 tesla was placed at the inlet of a product silo. The magnet was inserted in a stainless steel pipe to form a magnet bar. The magnet bars were arrayed in two lines to form the metal-scavenging apparatus. The iron content of the product granular bisphenol A sampled at the outlet of the product silo was 0.1 ppm by mass. After the bisphenol A was heated at 220°C for 40 minutes in the air, the color evaluated by visual observation using an APHA standard color was APHA 15.

### [Industrial Applicability]

The present invention provides a process for producing high-quality granular bisphenol A. In the process, can be efficiently removed metal powder that have come into the bisphenol A in a granulation step or a subsequent step thereof or metal powder that have resulted from regular or irregular overhaul inspection or repairing of various devices in the a production apparatus.
Further, can be also avoided a problem of substandard quality caused by metal powder in a polycarbonate production process that is a delivered site of the product granular bisphenol A or in a facility (receiving silo or the like) receiving the product granular bisphenol A in the polycarbonate production process.

## Claims

1. A process for producing high-quality granular bisphenol A, comprising:
contacting granular bisphenol A (2,2-bis(4-hydroxyphenyl)propane), to a metal-scavenging apparatus placed in a granulation step or a subsequent step thereof so as to collect metal powder contained in the granular bisphenol A and to reduce an iron content of the granular bisphenol A.

2. The process for producing high-quality granular bisphenol A according to claim 1, wherein
the metal-scavenging apparatus is placed in a pipe drawing out granular bisphenol A at the bottom of a granulation tower, in an inlet or an outlet of a product silo, or in an inlet or an outlet of a receiving silo for granular bisphenol A on a delivered site.

3. The process for producing high-quality granular bisphenol A according to claim 1 or 2, wherein
the metal powder is stainless-steel powder and/or iron powder.

4. The process for producing high-quality granular bisphenol A according to in any one of claims 1 to 3, wherein
the iron content of the granular bisphenol A after the granulation step is less than 1 ppm by mass.

5. The process for producing high-quality granular bisphenol A according to any one of claims 1 to 4, wherein
the metal-scavenging apparatus is provided with a magnet.

6. The process for producing high-quality granular bisphenol A according to claim 5, wherein
the surface magnetic flux density of the magnet is 0.7 tesla (T) or more.

7. The process for producing high-quality granular bisphenol A according to any one of claims 1 to 6, wherein
the metal-scavenging apparatus is a magnet bar placed perpendicularly to the flow direction of granular bisphenol A conveyed.
